# EUROPÄISCHE PATENTANMELDUNG

(11) **EP 0 846 752 A2**
(43) Veröffentlichungstag der Anmeldung: **10.06.1998**
(21) Anmeldenummer: 97120330.2
(22) Anmeldetag: 20.11.1997
(51) Int. Cl.: C11D 1/29, C11D 1/12, C11D 3/20, C11D 17/00, C11D 17/08, A61K 7/06, A61K 7/50

(54) **Fliessfähige, wässrige Perlglanzdispersion mit Behensäure als perlglanzgebender Komponente**

(30) Priorität: 03.12.1996 DE 19650089
(71) Anmelder: Clariant GmbH, 65929 Frankfurt am Main (DE)
(72) Erfinder: Turowski-Wanke, Angelika, Dr., 65777 Kelkheim (DE); Naumann, Peter, Dl., 65232 Taunusstein (DE)

(57) **Zusammenfassung**

Gegenstand der Erfindung ist ein fließfähiges Perlglanzkonzentrat mit einem Gehalt von 5 - 30% an Behensäure, formuliert in einer wäßrigen Dispersion, die gleichzeitig 1 - 50% an C₁₂- bis C₁₄-Ethersulfaten oder Acylisethionaten und gegebenenfalls 0,1 bis 10 % an mehrwertigen Alkoholen enthält. Die erfindungsgemäße Zusammensetzung ist in flüssigen Tensidformulierungen einsetzbar.

## Beschreibung

Diese Erfindung betrifft eine fließ- oder pumpfähige, wäßrige Perlglanzdispersion mit einem Gehalt von 5-30 % an perlglanzgebenden Komponenten.

Um Tensidformulierungen ein besseres Aussehen und damit auch einen höheren Handelswert zu geben werden oft Perlglanzdispersionen eingearbeitet. Beispiele hierfür sind flüssige Wasch- und Reinigungsmittel (z.B. Bodenreiniger, Geschirrspülmittel,) sowie flüssige kosmetische Präparate (z.B. Körperpflege- und Körperreinigungsmittel, Shampoos, Badezusätze, usw.). Perlglanzdispersionen verleihen den Formulierungen ein seiden- bzw. perlmuttartiges Aussehen. Der Effekt ensteht durch Lichtstreuung an den dispergierten, meist blättchenförmigen Kristallen der perlglanzgebenden Komponenten.

Perlglanzdispersionen nach dem Stand der Technik bestehen hauptsächlich aus mindestens einer perlglanzgebenden Verbindung, mindestens einem Dispergiermittel und Wasser. Perlglanzgebende Verbindungen sind beispielsweise Fettsäuremonoalkanolamide, Fettsäuredialkanolamide, Monoester oder Diester von Ethylenglykol oder Mischungen davon, Propylenglykol oder dessen Oligomere, Mono- oder Diester von Alkylenglykolen mit Fettsäuren, Fettsäuren und deren Metallsalze, Monoester oder Polyester von Glycerin mit Carbonsäuren und Ketosulfone verschiedener Art.

Perlglanzkonzentrate auf Basis der genannten Perlglanzbildner sind beispielsweise aus DE-A-16 69 152, JP-56/71021 (Chem. Abstr. 95/156360), DE-A-34 11 328 und DE-A-35 19 081 bekannt.

Die aus diesen Druckschriften bekannten Konzentrate enthalten als Teil der perlglanzbildenden Stoffe Fettsäuremonoalkanolamide oder Fettsäuredialkanolamide. Alkanolamide und deren Derivate werden aber verdächtigt, an der Bildung von Nitrosaminen beteiligt zu sein. Es ist daher erwünscht, kosmetische Zubereitungen ohne solche Alkanolamide und Alkanolamid-Derivate zu formulieren.

Ein Weglassen der Fettsäurealkanolamide aus den bekannten Perlglanz-Konzentraten führt aber zu einer deutlichen Verringerung der perlglanzgebenden Eigenschaften. Es wurde daher schon in DE-37 24 547 vorgeschlagen als perlglanzbildenden Stoff im wesentlichen lineare, gesättigte Fettsäuren, unter anderem auch Behensäure, einzusetzen.

In EP-0 449 904 wird offenbart, daß man, um einen befriedigenden Perlglanz im Endprodukt zu erhalten, deutlich höhere Konzentrationen an perlglanzgebenden Stoffen einsetzten muß. Des weiteren wird beschrieben, daß Perlglanzmittel basierend auf Fettsäuren, deren Salzen sowie deren Estern als perlglanzgebende Komponente nur eine geringe Wärmestabilität aufweisen und bei üblichen Einsatzkonzentrationen in Tensidrezepturen im Laufe der Lagerung angelöst bis aufgelöst werden. Diese Nachteile haben dazu geführt, daß sich solche Systeme auf dem Markt nicht durchgesetzt haben.

Es bestand daher die Aufgabe, ein Perlglanzmittel zu entwickeln, das ohne Feftsäurealkanoamide oder Fettsäureester auskommt, eine gute Lagerstabilität aufweist, und auch einen dem Stand der Technik entsprechenden Perlglanzeffekt hervorruft.

Überraschend wurde jetzt gefunden, daß Perlglanz-Konzentrate mit Behensäure als perlglanzgebender Komponente in Verbindung mit einem Alkalisalz von Laurylethersulfat als Dispergiermittel innerhalb von 1-5 Tagen zu einem ausgezeichneten Perlglanz-Effekt führen.

Gegenstand der Erfindung ist eine Perlglanzdispersion, enthaltend
A) 5 - 30 Gew.-% Behensäure und/oder Behensäuresalze
B1) 1 bis 50 Gew.-% einer oder mehrerer Verbindungen der Formel

   R¹ - O - (C₂H₄O)ₙ - SO₃^{⊖} X⁺

   worin
   - R¹: C₁₂- bis C₁₄-Alkyl,
   - n: 2 oder 3 und
   - X⁺: ein Alkalimetallion oder ein Ammoniumion bedeuten, oder
B2) 1 bis 50 Gew.-% einer oder mehrerer Verbindungen der Formel

   R² - COO - CH₂ - CH₂ - SO₃^{⊖} X⁺

   worin
   - R²: C₈- bis C₁₈-Alkyl,
   - X⁺: ein Alkalimetallion oder ein Ammoniumion bedeuten, oder
B3) 1 bis 50 Gew.-% einer Mischung der unter B1) und B2) genannten Stoffe,
   und
C) Wasser und gegebenenfalls weitere übliche Inhaltsstoffe ad 100 Gew.-%
   X bedeutet bevorzugt Natrium oder Kalium, n bedeutet bevorzugt 2. Bevorzugt werden 10 bis 20 Gew.-% Behensäure verwendet. Bevorzugt werden von den unter B1) genannten Stoffen mehr als 10 bis 50 Gew.-% verwendet, insbesondere mehr als 10 bis 30 Gew.-%. Bevorzugt werden von den unter B2) genannten Stoffen 1 bis 20 Gew.-% verwendet, insbesondere 2 bis 16 Gew.-%. Als Behensäuresalze werden bevorzugt Alkali- und Ammoniumsalze verwendet, insbesondere Natrium- und Kaliumsalze.

Die erfindungsgemäßen Perlglanzdispersionen können als weitere übliche Inhaltsstoffe bis zu 10 Gew.-% an mehrwertigen C₂- bis C₈-Alkoholen enthalten. Als mehrwertige Alkohole sind C₂- bis C₆-Polyole bevorzugt, insbesondere Ethylenglykol, 1,2- und 1,3-Propylenglykol, Glycerin, Di- und Triethylenglykol, Erythrit, Arabit, Adonit, Xylit, Sorbit, Mannit und Dulcit. Weitere übliche Inhaltsstoffe sind auch Konservierungsmittel und Puffersubstanzen. Für den Fall, daß die Perlglanzdispersion nur eine Verbindung der Formel B1) enthält, ist die Dispersion vorzugsweise frei von mehrwertigen Alkoholen.

Die erfindungsgemäßen wäßrigen Perlglanzdispersionen sind fließ- und pumpfähig und damit auf automatischen Pump-, Dosier- und Mischanlagen ohne Schwierigkeiten einsetzbar. Sie haben gegenüber den Dispersionen des Standes der Technik folgende Vorteile:
- sie zeigen ein besseres Schaumverhalten im Ross-Miles Test
- es werden keine Glycol- oder Ethylenglycolstearate bzw. -ester eingesetzt
- sie können ohne Alkohole hergestellt werden
- sie sind frei von nitrosierbaren Aminen bzw. Alkanolamiden
- sie stellen bei der Herstellung geringere Anforderungen an Prozeßführung und Apparatur
- die erhaltenen Perlglanz-Konzentrate sind dünnflüssig und damit sehr gut pumpbar. Die geringe Viskosität wirkt sich außerdem günstig auf die Wärme/Kälteverhalten aus.

Die Herstellung der erfindungsgemäßen Perlglanzdispersionen erfolgt bevorzugt in der Weise, daß Wasser und der Dispergator zunächst in einem Mischgefäß (heizbarer Kessel) auf eine Temperatur von etwa 70 bis etwa 90°C erwärmt werden. Getrennt von diesem Vorgang wird die Behensäure in einem zweiten Kessel bei einer Temperatur von im allgemeinen etwa 70 bis etwa 90°C homogen aufgeschmolzen. Die homogen aufgeschmolzene Behensäure wird bei der angegebenen Temperatur von etwa 70 bis etwa 90°C unter Rühren in die Mischung aus Wasser und Dispergator eingebracht. Die erhaltene Dispersion wird zur Temperierung und Homogenisierung bei einer Temperatur von etwa 70 bis etwa 90°C unter Rühren gehalten (etwa 15 bis 30 Minuten). Unter Weiterrühren wird die Dispersion schrittweise auf eine Temperatur von 25°C abgekühlt. Die Viskosität des Perlglanzkonzentrats ist meistens so niedrig, daß auf den Einsatz besonderer Rühraggregate wie Homogenisatoren oder andere hochtourige Mischvorrichtungen verzichtet werden kann. Während des Abkühlens kristallisiert die Behensäure aus, bei 25°C kann das dünnflüssige Perlglanz-Konzentrat abgefüllt werden.

Die erfindungsgemäßen Perlglanzdispersionen eignen sich zur Herstellung perlglänzender flüssiger, wäßriger Tensidformulierungen. Sie können beispielsweise in flüssige Feinwaschmittel, Universalwaschmittel, Handgeschirrspülmittel, Klarspüler, flüssige Reinigungs- und Desinfektionsmittel, flüssige Seifen, Haarshampoos, Haarspülungen, Haarfärbemittel, Haarwellmittel, Schaumbäder, Gesichtsreinigungsmittel, Duschbadzubereitungen und 2 in 1-Formulierungen eingearbeitet werden. Die erfindungsgemäßen Perlglanzdispersionen unterstützen den Konditionierungseffekt von Haarshampoos, insbesondere von sogenannten 2 in 1 Shampoos, die wasserlösliche und/oder nicht wasserlösliche dispergierte Agenzien enthalten, wie etwa Siliconderivate (z.B. Dimethicone).

Die effindungsgemäßen wäßrigen Perlglanzdispersionen lassen sich in Tensidformulierungen leicht kalt einarbeiten, wodurch diese den angestrebten Perlglanz erhalten. Die erforderliche Menge an Perlglanzdispersion liegt bei 1 bis 10 Gew.-%, vorzugsweise 2 bis 5 Gew.-%, bezogen auf das Gewicht der zu behandelnden Tensidformulierung. Die Perlglanzdispersion wird in der angegebenen Menge den Tensidformulierungen vorzugsweise als letzte Komponente, bei Raumtemperatur unter Rühren, zugesetzt. Das Perlglanzkonzentrat entfaltet seinen Glanz und seine Ergiebigkeit in der Formulierung nach ca. 1-5 Tagen. Danach werden Endprodukte erhalten, die einen ausgezeichneten stabilen Perlglanz aufweisen.

Die tensidhaltigen Zusammensetzungen, in denen die erfindungsgemäßen Perlglanzdispersionen verwendet werden können, werden im folgenden beschrieben.

Als anionische Tenside kommen Sulfonate, Sulfate, Carboxylate, Phosphate und Mischungen aus den genannten Verbindungen in Betracht. Geeignete Kationen sind hierbei Alkalimetalle, wie z.B. Natrium oder Kalium oder Erdalkalimetalle, wie z.B. Calcium oder Magnesium sowie Ammonium, substituierte Ammoniumverbindungen, einschließlich Mono-, Di- oder Triethanolammoniumkationen und Mischungen der Kationen. Folgende Typen von anionischen Tensiden sind von besonderem Interesse: Alkylestersulfonate, Alkylsulfate, Alkylethersulfate, Alkylbenzolsulfonate, sekundäre Alkansulfonate, Seifen wie im folgenden beschrieben.

Alkylestersulfonate sind unter anderem lineare Ester von C₈-C₂₀-Carboxylsäuren (d.h. Fettsäuren), welche mittels gasförmigem SO₃ sulfoniert werden, wie in "The Journal of the American Oil Chemists Society" 52(1975), pp. 323-329 beschrieben wird. Geeignete Ausgangsmaterialien sind natürliche Fette wie z.B. Talg, Palmöl oder Cocosöl, können aber auch synthetischer Natur sein. Bevorzugte Alkylestersulfonate, speziell für Waschmittelanwendungen, sind Verbindungen der Formel 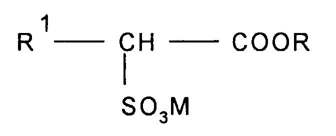 worin R¹ einen C₈-C₂₀-Kohlenwasserstoffrest, bevorzugt Alkyl und R einen C₁-C₆ Kohlenwasserstoffrest, bevorzugt Alkyl, darstellt. M steht für ein Kation, das ein wasserlösliches Salz mit dem Alkylestersulfonat bildet. Geeignete Kationen sind Natrium, Kaum, Lithium oder Ammoniumkationen, wie Monoethanolamin, Diethanolamin und Triethanolamin. Bevorzugt bedeuten R¹ C₁₀-C₁₆-Alkyl und R Methyl, Ethyl oder Isopropyl. Besonders bevorzugt sind Methylestersulfonate, in denen R¹ C₁₀-C₁₆-Alkyl bedeutet.

Alkylsulfate sind hier wasserlösliche Salze oder Säuren der Formel ROSO₃M, worin R bevorzugt ein C₁₀-C₂₄-Kohlenwasserstoffrest, bevorzugt C₁₀-C₂₀-Alkyl oder Hydroxyalkyl, besonders bevorzugt C₁₂-C₁₈ Alkyl- oder Hydroxyalkyl darstellt. M ist Wasserstoff oder ein Kation, z.B. ein Alkalimetallkation (z.B. Natrium, Kaum, Lithium), Ammonium oder substituiertes Ammonium z.B. Methyl-, Dimethyl- und Trimethylammoniumkationen und quaternäre Ammoniumkationen, wie Tetramethylammonium- und Dimethylpiperidiniumkationen und quaternäre Ammoniumkationen, abgeleitet von Alkylaminen wie Ethylamin, Diethylamin, Triethylamin und Mischungen davon. Alkylketten mit C₁₂-C₁₆ sind für niedrige Waschtemperaturen (z.B. unter ca. 50°C) und Alkylketten mit C₁₆-C₁₈ für höhere Waschtemperaturen (z.B. oberhalb ca. 50°C) bevorzugt.

Alkylethersulfate sind wasserlösliche Salze oder Säuren der Formel RO(A)ₘ SO₃M, worin R einen unsubstituierten C₁₀-C₂₄-Alkyl- oder Hydroxyalkylrest, bevorzugt einen C₁₂-C₂₀ Alkyl- oder Hydroxyalkylrest, besonders bevorzugt C₁₂-C₁₈-Alkyl- oder Hydroxyalkylrest darstellt. A ist eine Ethoxy- oder Propoxyeinheit, m ist eine Zahl größer als 0, vorzugsweise zwischen ca. 0,5 und ca. 6, besonders bevorzugt zwischen ca. 0,5 und ca. 3 und M ist ein Wasserstoffatom oder ein Kation wie z.B. Natrium, Kalium, Lithium, Calcium, Magnesium, Ammonium oder ein substituiertes Ammoniumkation. Spezifische Beispiele von substituierten Ammoniumkationen sind Methyl-, Dimethyl-, Trimethylammonium- und quaternäre Ammoniumkationen wie Tetramethylammonium und Dimethylpiperidiniumkationen, sowie solche, die von Alkylaminen, wie Ethylamin, Diethylamin, Triethylamin, Mischungen davon abgeleitet sind. Als Beispiele seien C₁₂-C₁₈-Fettalkoholethersulfate genannt, wobei der Gehalt an Ethylenoxid 1, 2, 2.5, 3 oder 4 mol pro mol Fettalkoholethersulfat beträgt, und in denen M Natrium oder Kalium ist.

In sekundären Alkansulfonaten kann die Alkylgruppe entweder gesättigt oder ungesättigt, verzweigt oder linear und gegebenenfalls mit einer Hydroxylgruppe substituiert sein. Die Sulfogruppe kann eine beliebige Position an der gesamten C-Kette einnehmen, wobei die primären Methylgruppen am Kettenanfang und Kettenende keine Sulfogruppen besitzen. Die bevorzugten sekundären Alkansulfonate enthalten lineare Alkylketten mit ca. 9 bis 25 Kohlenstoffatomen, bevorzugt von ca. 10 bis ca. 20 Kohlenstoffatome und besonders bevorzugt ca. 13 bis 17 Kohlenstoffatome. Als Kation ist beispielsweise Natrium, Kaum, Ammonium, Mono-, Di- oder Triethanolammonium, Calcium oder Magnesium bevorzugt.

Weitere geeignete anionische Tenside sind Alkenyl- oder Alkylbenzolsulfonate. Die Alkenyl- oder Alkylgruppe kann verzweigt oder linear und gegebenenfalls mit einer Hydroxylgruppe substituiert sein. Die bevorzugten Alkylbenzolsulfonate enthalten lineare Alkylketten mit ca. 9 bis 25 Kohlenstoffatomen, bevorzugt von ca. 10 bis ca. 13 Kohlenstoffatome, das Kation ist Natrium, Kalium, Ammonium, Mono-, Di- oder Triethanolammonium, Calcium oder Magnesium und Mischungen davon. Für milde Tensidsysteme ist Magnesium als Kation bevorzugt, für Standardwaschanwendungen dagegen Natrium. Gleiches gilt für Alkenylbenzolsulfonate

Der Begriff anionische Tenside schließt auch Olefinsulfonate mit ein, die durch Sulfonierung von C₁₂-C₂₄-, vorzugsweise C₁₄-C₁₆-α-Olefinen mit Schwefeltrioxid und anschließende Neutralisation erhalten werden. Bedingt durch das Herstellverfahren, können diese Olefnsulfonate kleinere Mengen an Hydroxyalkansulfonaten und Alkandisulfonaten enthalten. Spezielle Mischungen von α-Olefinsulfonaten sind in US-3,332,880 beschrieben.

Weitere bevorzugte anionische Tenside sind Carboxylate, z.B. Fettsäureseifen und vergleichbare Tenside. Die Seifen können gesättigt oder ungesättigt sein und können verschiedene Substituenten, wie Hydroxylgruppen oder α-Sulfonatgruppen enthalten. Bevorzugt sind lineare gesättigte oder ungesättigte Kohlenwasserstoffreste als Anteil mit ca. 6 bis ca. 30, bevorzugt ca. 10 bis ca. 18 Kohlenstoffatomen.

Als anionische Tenside kommen weiterhin Salze von Acylaminocarbonsäuren in Frage, die durch Umsetzung von Fettsäurechloriden mit Natriumsarkosinat im alkalischen Medium entstehenden Acylsarcosinate; Fettsäure-EiweißKondensationsprodukte, die durch Umsetzung von Fettsäurechloriden mit Oligopeptiden erhalten werden; Salze von Alkylsulfamidocarbonsäuren, Salze von Alkyl- und Alkylarylethercarbonsäuren; C₈-C₂₄-Olefinsulfonate, sulfonierte Polycarboxylsäuren, hergestellt durch Sulfonierung der Pyrolyseprodukte von Erdalkalimetallcitraten, wie z.B. beschrieben in GB-1,082,179; Alkylglycerinsulfate, Fettacylglycerinsulfate, Alkylphenolethersulfate, primäre Paraffinsulfonate, Alkylphosphate, Alkyletherphosphate, Isethionate, wie Acylisethionate, N-Acyltauride, Alkylsuccinate, Sulfosuccinate, Monoester der Sulfosuccinate (besonders gesättigte und ungesättigte C₁₂-C₁₈-Monoester) und Diester der Sulfosuccinate (besonders gesättigte und ungesättigte C₁₂-C₁₈-Diester), Acylsarcosinate, Sulfate von Alkylpolysacchariden wie Sulfate von Alkylpolyglycosiden, verzweigte primäre Alkylsulfate und Alkylpolyethoxycarboxylate wie die der Formel RO(CH₂CH₂)ₖCH₂COO⁻M⁺, worin R C₈-C₂₂-Alkyl, k eine Zahl von 0 bis 10 und M ein Kation ist, Harzsäuren oder hydrierte Harzsäuren, wie Rosin oder hydriertes Rosin oder Tallölharze und Tallölharzsäuren. Weitere Beispiele sind in "Surface Active Agents and Detergents" (Vol. I und II, Schwartz, Perry und Berch) beschrieben.

Als nicht-ionische Tenside kommen beispielsweise folgende Typen in Frage:

Polyethylen-, Polypropylen- und Polybutylenoxidkondensate von Alkylphenolen.

Diese Verbindungen umfassen die Kondensationsprodukte von Alkylphenolen mit einer C₆-C₂₀-Alkylgruppe, die entweder linear oder verzweigt sein kann, mit Alkenoxiden. Bevorzugt sind Verbindungen mit ca. 5 bis 25 mol Ethylenoxid pro mol Alkylphenol. Kommerziell erhältliche Tenside diesen Typs sind z.B. Igepal® CO-630, Triton® X-45, X-114, X-100 und X102, und die ®Arkopal-N-Marken der Hoechst AG.

Kondensationsprodukte von aliphatischen Alkoholen mit ca. 1 bis ca. 25 mol Ethylenoxid.

Die Alkylkette der aliphatischen Alkohole kann linear oder verzweigt, primär oder sekundär sein, und enthält im allgemeinen ca. 8 bis ca. 22 Kohlenstoffatome. Besonders bevorzugt sind die Kondensationsprodukte von C₁₀-C₂₀-Alkoholen, mit ca. 2 bis ca. 18 mol Ethylenoxid pro mol Alkohol. Die Alkylkette kann gesättigt oder auch ungesättigt sein. Die Alkoholethoxilate können eine enge ("Narrow Range Ethoxilates") oder eine breite Homologenverteilung des Ethylenoxides ("Broad Range Ethoxylates") aufweisen. Beispiele von kommerziell erhältlichen nichtionischen Tensiden dieses Types sind Teritol® 15-S-9 (Kondensationsprodukt eines C₁₁-C₁₅ linearen sekundären Alkohols mit 9 mol Ethylenoxid), Tergitol® 24-L-NMW (Kondensationsprodukt eines C₁₂-C₁₄-linearen primären Alkohols mit 6 mol Ethylenoxid mit enger Molgewichtsverteilung). Ebenfalls unter diese Produktklasse fallen die Genapol®-Marken der Hoechst AG.

Kondensationsprodukte von Ethylenoxid mit einer hydrophoben Basis, gebildet durch Kondensation von Propylenoxid mit Propylenglykol.

Der hydrophobe Teil dieser Verbindungen weist bevorzugt ein Molekulargewicht zwischen ca. 1500 und ca. 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhätliche Beispiele dieser Produktklasse sind die Pluronic®-Marken der BASF und die ®Genapol PF-Marken der Hoechst AG.

Kondensationsprodukt von Ethylenoxid mit einem Reaktionsprodukt von Propylenoxid und Ethylendiamin.

Die hydrophobe Einheit diese Verbindungen besteht aus dem Reaktionsprodukt von Ethylendiamin mit überschüssigem Propylenoxid und weist im allgemeinen ein Molekulargewicht von ca. 2500 bis ca. 3000 auf. An diese hydrophobe Einheit wird Ethylenoxid addiert, bis das Produkt einen Gehalt von ca. 40 bis ca. 80 Gew.-% Polyoxyethylen und ein Molekulargewicht von ca. 5000 bis ca. 11000 aufweist. Kommerziell erhältliche Beispiele dieser Verbindungsklase sind die ®Tetronic-Marken der BASF und die ®Genapol PN-Marken der Hoechst AG.

### Semipolare nichtionische Tenside

Diese spezielle Kategorie von nichtionischen Verbindungen umfaßt wasserlösliche Aminoxide, wasserlösliche Phosphinoxide und wasserlösliche Sulfoxide jeweils mit einem Alkylrest von ca. 10 bis ca. 18 Kohlenstoffatomen. Semipolare nichtionische Tenside sind auch Aminoxide der Formel

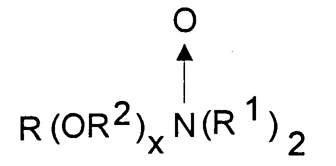

R ist hierbei eine Alkyl-Hydroxyalkyl- oder Alkylphenolgruppe mit jeweils ca. 8 bis ca. 22 Kohlenstoffatomen, R² ist eine Alkylen- oder Hydroxyalkylengruppe mit ca. 2 bis 3 Kohlenstoffatomen oder Mischungen hiervon, jeder Rest R¹ ist eine Alkyl- oder Hydroxyalkylgruppe mit ca. 1 bis ca. 3 Kohlenstoffatomen oder eine Polyethylenoxidgruppe mit ca. 1 bis ca. 3 Ethylenoxideinheiten. Die R¹-Gruppen können miteinander über ein Sauerstoff- oder Stickstoffatom verbunden sein und somit einen Ring bilden. Aminoxide dieser Art sind besonders C₁₀-C₁₈-Alkyldimethylaminoxide und C₈-C₁₂-Alkoxiethyl-Dihydroxyethylaminoxide.

### Fettsäureamide

Fettsäureamide besitzen die Formel 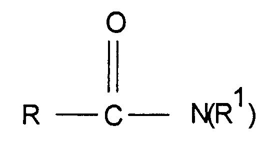 worin R eine Alkylgruppe mit ca. 7 bis ca. 21, bevorzugt ca. 9 bis ca. 17 Kohlenstoffatomen ist und jeder Rest R¹ Wasserstoff, C₁-C₄-Alkyl, C₁-C₄-Hydroxyalkyl oder (C₂H₄O)ₓH bedeutet, wobei x von ca. 1 bis ca. 3 variiert. Bevorzugt sind C₈-C₂₀-Amide, -monoethanolamide, -diethanolamide und -isopropanolamide.

Weitere geeignete nicht-ionische Tenside sind insbesondere Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, alkoxylierte Triglycamide, Mischether oder Mischformyle, Fettsäure-N-alkylglucamide, Proteinhydrolysate, Phosphinoxide oder Dialkylsulfoxide.

Typische Beispiele für amphotere bzw. zwitterionische Tenside sind Alkylbetaine, Alkylamidbetaine, Aminopropionate, Aminoglycinate, oder amphotere Imdiazolinium-Verbindungen der Formel 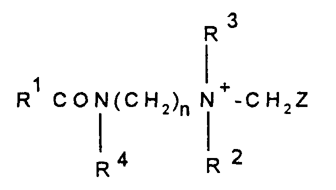 worin R¹ C₈-C₂₂-Alkyl- oder -Alkenyl, R² Wasserstoff oder CH₂CO₂M, R³ CH₂CH₂OH oder CH₂CH₂OCH₂CH₂COOM, R⁴ Wasserstoff, CH₂CH₂OH oder CH₂CH₂COOM, Z CO₂M oder CH₂CO₂M, n 2 oder 3, bevorzugt 2, M Wasserstoff oder ein Kation wie Alkalimetall, Erdalkalimetall, Ammoniak oder Alkanolammonium bedeutet.

Bevorzugte amphotere Tenside dieser Formel sind Monocarboxylate und Dicarboxylate. Beispiele hierfür sind Cocoamphocarboxypropionat, Cocoamidocarboxypropionsäure, Cocoamphocarboxyglycinat (auch als Cocoamphodiacetat bezeichnet) und Cocoamphoacetat.

Weitere bevorzugte amphotere Tenside sind Alkyldimethylbetaine und Alkyldipolyethoxybetaine mit einem Alkylrest, der linear oder verzweigt sein kann, mit ca. 8 bis ca. 22 Kohlenstoffatomen, bevorzugt mit 8 bis 18 Kohlenstoffatomen und besonders bevorzugt mit ca. 12 bis ca. 18 Kohlenstoffatomen. Diese Verbindungen werden z.B. von der Hoechst AG unter dem Handelnamen ®Genagen LAB vermarktet.

Als kationische Tenside werden quartäre Ammoniumsalze vom Typ 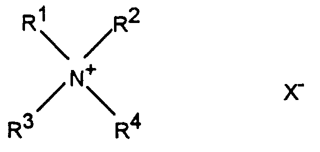 eingesetzt, worin
- R¹: = C₈-C₂₄ n-, bzw. iso-Alkyl, bevorzugt C₁₀-C₁₈ n-Alkyl
- R²: = C₁-C₄-Alkyl, bevorzugt Methyl
- R³: = R¹ oder R²
- R⁴: = R² oder Hydroxyethyl oder Hydroxypropyl oder deren Oligomere
- X⁻: = ein geeignetes Anion
sind.

Beispiele hierfür sind Distearyldimethylammoniumchlorid, Ditalgalkyldimethylammoniumchlorid, Ditalgalkylmethylhydroxypropylammoniumchlorid, Cetyltrimethylammoniumchlorid oder auch die entsprechenden Benzylderivate wie etwa Dodecyldimethylbenzylammoniumchlorid. Cyclische quartäre Ammoniumsalze, wie etwa Alkyl-Morpholinderivate können ebenfalls verwendet werden.

Darüberhinaus können neben den quartären Ammoniumverbindungen Imidazolinium-Verbindungen (1) und Imidazolinderivate (2) eingesetzt werden. 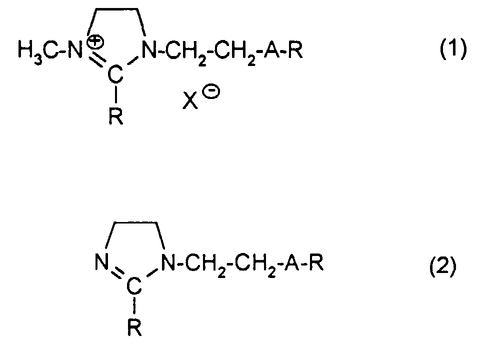 worin
- R: = C₈-C₂₄ n-, bzw. iso-Alkyl, bevorzugt C₁₀-C₁₈ n-Alkyl
- X: = Bromid, Chlorid, Jodid, Methosulfat
- A: = -NH-CO-, -CO-NH-, -O-CO-, -CO-O-
ist.

Weitere geeignete kationische Tenside sind die sogenannten Esterquats. Es handelt sich hierbei um Umsetzungsprodukte von Alkanolaminen und Fettsäuren, die anschließend mit üblichen Alkylierungs- oder Hydroxyalkylierungsagenzien quaterniert werden.

Bevorzugt als Alkanolamine sind Verbindungen gemäß der Formel 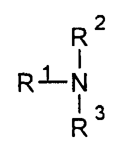 mit
- R¹: = C₁-C₃ Hydroxyalkyl, bevorzugt Hydroxyethyl und
- R², R3: = unabhängig voneinander R¹ oder C₁-C₃ Alkyl, bevorzugt Methyl.

Besonders bevorzugt sind Triethanolamin und Methyldiethanolamin.

Weitere besonders bevorzugte Ausgangsprodukte für Esterquats sind Aminoglycerinderivate, wie z. B. Dimethylaminopropandiol.

Alkylierungs- bzw. Hydroxyalkylierungsagenzien sind Alkylhalogenide, bevorzugt Methylchlorid, Dimethylsulfat, Ethylenoxid und Propylenoxid.

Beispiele für Esterquats sind Verbindungen der Formeln: 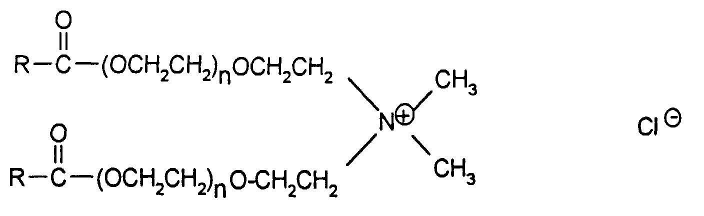 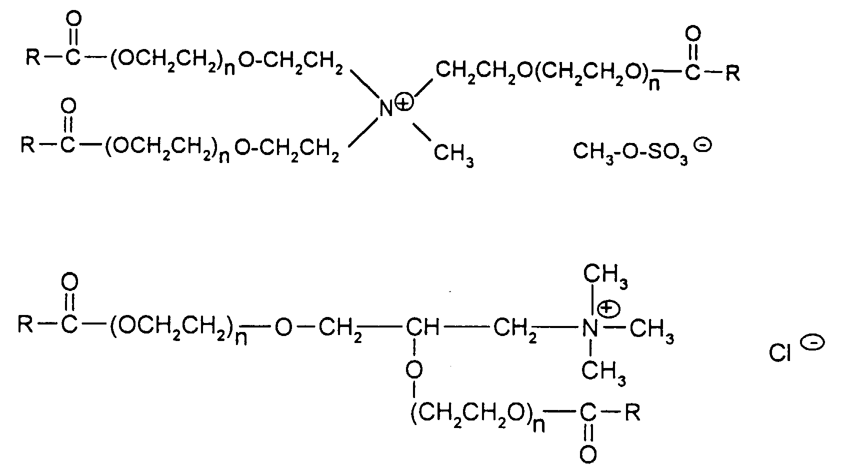 wobei R-C-O abgeleitet ist von C₈-C₂₄-Fettsäuren, die gesättigt oder ungesättigt sein können. Beispiele hierfür sind Capronsäure, Caprylsäure, hydrierte oder nicht oder nur teilweise hydrierte Talgfettsäuren, Stearinsäure, Olsäure, Linolensäure, Behensäure, Palminstearinsäure, Myristinsäure und Elaidinsäure. n liegt im Bereich von 0 bis 10, vorzugsweise 0 bis 3, besonders bevorzugt 0 bis 1.

### Beispiele

Die erfindungsgemäßen Perlglanzdispersionen werden in den folgenden Beispielen beschrieben. Die Mengen sind in Gewichtsprozent angegeben. Das Herstellen der Perlglanzdispersionen erfolgte nach der oben beschriebenen Arbeitsweise. Die erhaltenen wäßrigen Dispersionen wurden auf ihr Fließverhalten nach 3 Tagen und auf ihren Perlglanz in einer Shampoo-Formulierung getestet. Beide Tests erfolgten rein visuell im Vergleich zu Handelsprodukten. Die Testergebnisse sind in der folgenden Tabelle aufgeführt.

Als Vergleichsbeispiel (in der Tabelle "Vergleich" genannt) diente Euperlan® PK 3000 von Henkel. Es wurde 5%ig in eine Shampoo-Formulierung eingearbeitet, ebenso wie die erfindungsgemäßen Beispiele in der Tabelle.

Euperlan® PK 3000 ist ein Perlglanzmittel in dem Monoglykolmono- und distearat als perlglanzgebende Komponenten eingesetzt werden. Es enthält außerdem noch Alkylamidpropylbetain, Alkoholoxethylate und Kochsalz.

Die Mengenangaben für die Handelsprodukte in den nachfolgenden Tabellen I und II beziehen sich auf Reinsubstanzen, und nicht auf die handelsüblichen verdünnten Lösungen.

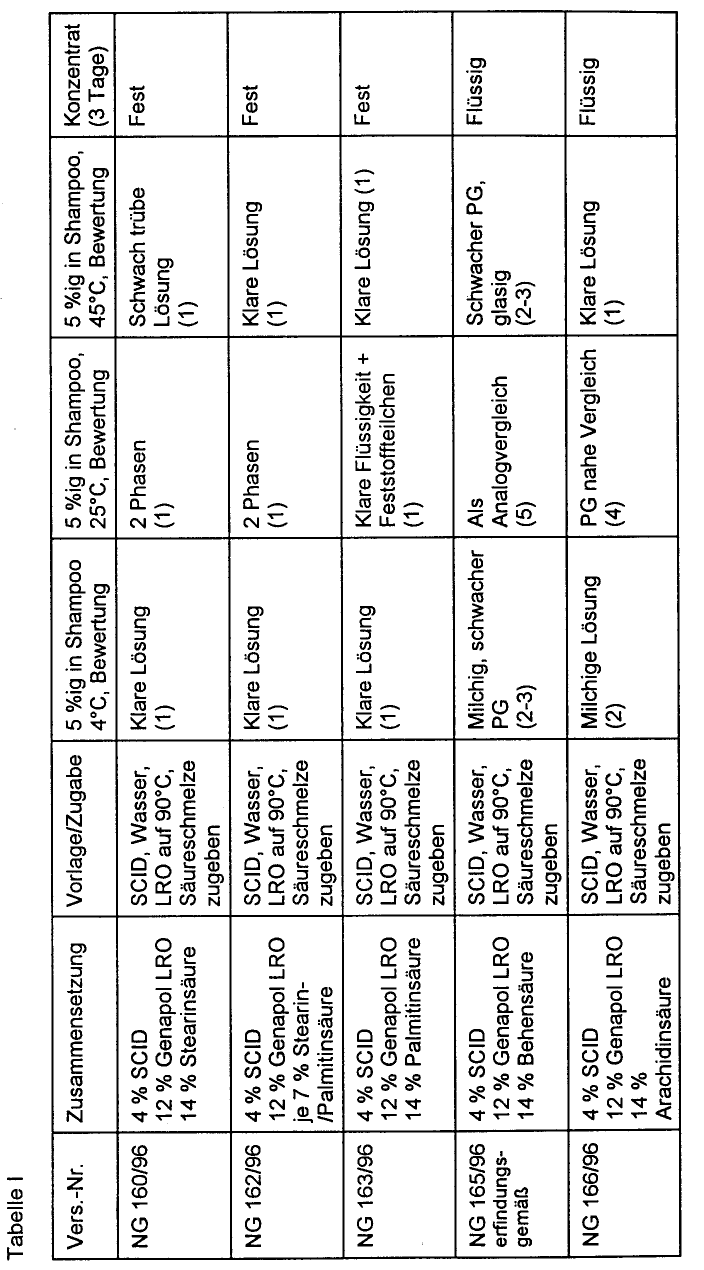

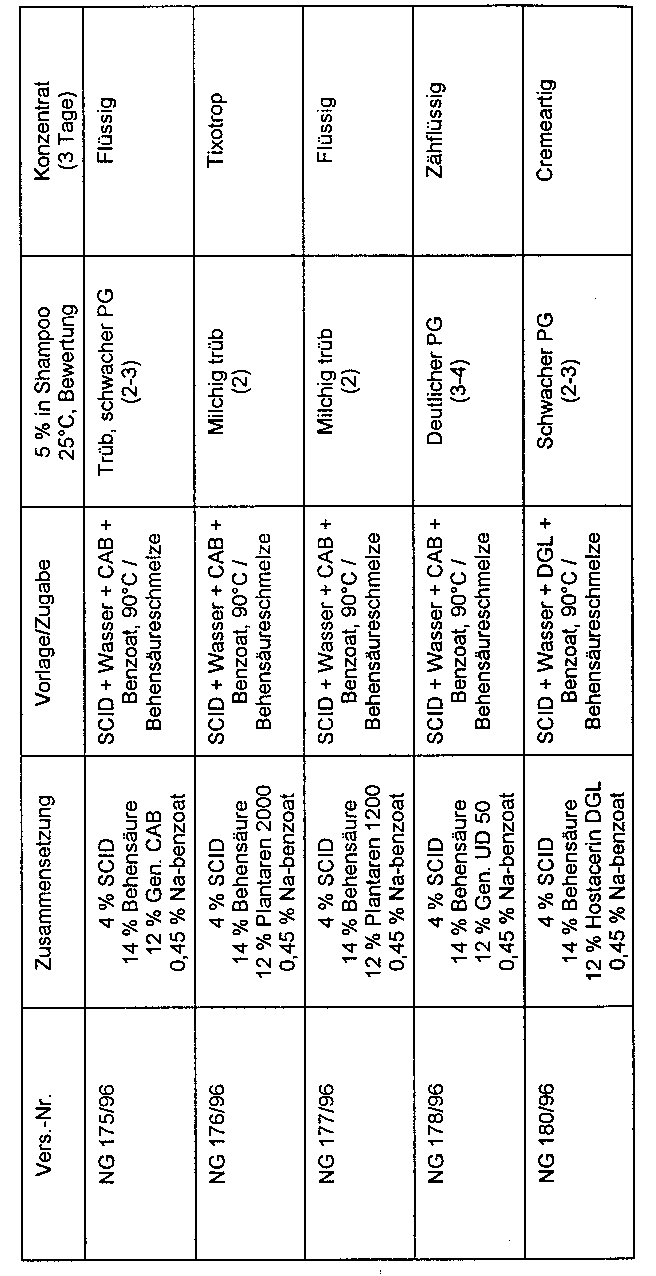

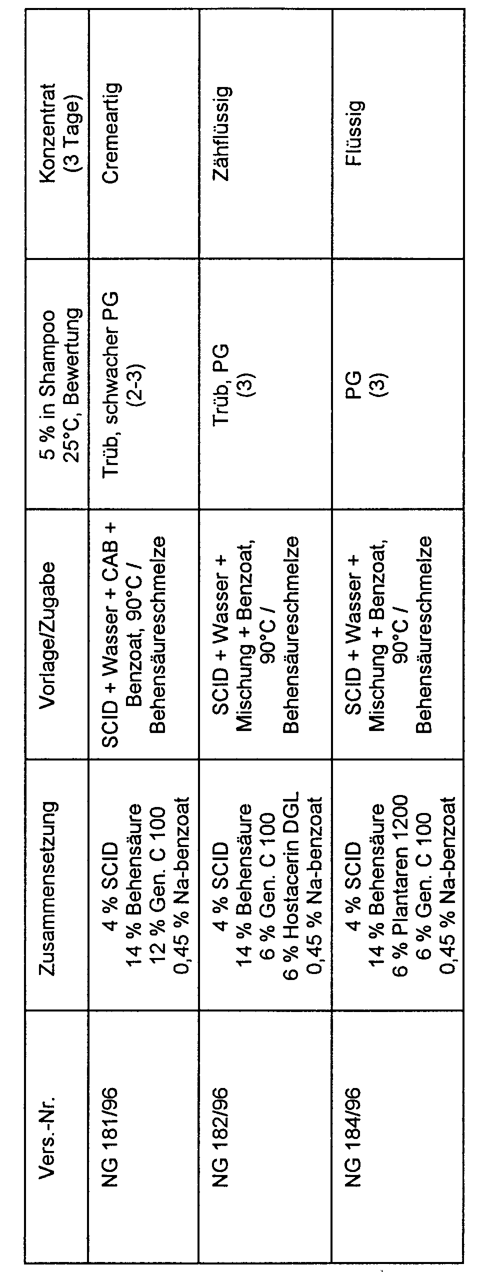

**Tabelle II**

| Vers.-Nr. | % Genapol LRO | % Behensäure (85 %ig) | % SCID | PG | Viskosität 20°C (mPas), 7. Woche |
|---|---|---|---|---|---|
| NG 2/97 | 12 | 14 | 1 | 4-5 | 1530 |
| NG 3/97 | 12 | 14 | 2 | 5 | 1740 |
| NG 4/97 | 12 | 14 | 3 | 5 | 1850 |
| NG 6/97 | 14 | 14 | 1 | 4-5 | 1250 |
| NG 7/97 | 14 | 14 | 2 | 5 | 1110 |
| NG 12/97 | 16 | 14 | 1 | 4-5 | 1760 |
| NG 15/97 | 16 | 14 | 2 | 5 | 19800 |
| NG 16/97 | 16 | 14 | 3 | 5 | 18000 (35 Tage) |
| NG 20/97 | 18 | 14 | 1 | 4 | 20000 |
| NG 33/97 | 20 | 14 | 1 | 3 | 18000(35 Tage) |
| NG 43/97 | 12 | 16 | 1 | 4 | 6250 |
| NG 44/97 | 12 | 16 | 2 | 4 | 5550 |
| NG 45/97 | 12 | 16 | 3 | 4 | 7800 |

Bewertungsstufen für den Perlglanz
- 1: = Klare Lösung
- 2: = Trüb, ohne Perlglanz
- 3: = Schwacher Perlglanz
- 4: = Perlglanz nahe Vergleich
- 5: = Perlglanz analog Vergleich

Gen. CAB = Genapol® CAB = Alkylamidopropylbetain (30 %ig)
Gen. UD 50 = Genapol® UD 050 = C11-Oxoalkoholpolyglykolether + 5 EO
Gen. C 100 = Genapol® C 100 = C 10/18-Fettalkoholpolyglykolether + 10 EO
Genapol LRO = 28% Alkylethersulfat-Natriumsalz in Wasser
Hostacerin® DGL = Oxethylierter Fettsäurepolyglycerinester
Plantaren® 1200 und 2000 = Alkylpolyglykoside der Firma Henkel
SCID = Hostapon® SCID = Acylisethionat
PG = Perlglanz

## Patentansprüche

1. Perlglanzdispersion, enthaltend
A) 5 - 30 Gew.-% Behensäure und/oder Behensäuresalze
B1) 1 bis 50 Gew.-% einer oder mehrerer Verbindungen der Formel
R¹ - O - (C₂H₄O)ₙ - SO₃^{⊖} X⁺
worin
R¹ C₁₂- bis C₁₄-Alkyl,
n 2 oder 3 und
X⁺ ein Alkalimetallion oder ein Ammoniumion bedeuten, oder
B2) 1 bis 50 Gew.-% einer oder mehrerer Verbindungen der Formel
R² - COO - CH₂ - CH₂ - SO₃^{⊖} X⁺
worin
R² C₈- bis C₁₈-Alkyl,
X⁺ ein Alkalimetallion oder ein Ammoniumion bedeuten, oder
B3) 1 bis 50 Gew.-% einer Mischung der unter B1) und B2) genannten Stoffe, und
C) Wasser und gegebenenfalls weitere übliche Inhaltsstoffe ad 100 Gew.-%

2. Perlglanzdispersion nach Anspruch 1, dadurch gekennzeichnet, daß n = 2 ist.

3. Perlglanzdispersion nach Anspruch 1 und/oder 2, dadurch gekennzeichnet, daß X Natrium oder Kalium bedeutet.

4. Perlglanzdispersion nach einem oder mehreren der Ansprüche 1 bis 3, dadurch gekennzeichnet, daß 10 bis 20 Gew.-% Behensäure verwendet werden.

5. Perlglanzdispersion nach einem oder mehreren der Ansprüche 1 bis 4, dadurch gekennzeichnet, daß in ihr die unter B1) und B2) genannten Verbindungen unabhängig voneinander in Mengen von 1 bis 20, insbesondere 2 bis 16 Gew.-% enthalten sind.

6. Perlglanzdispersion nach einem oder mehreren der Ansprüche 1 bis 3, dadurch gekennzeichnet, daß sie frei von mehrwertigen C₂-C₆-Alkoholen sind und mehr als 10 bis 50 Gew.-% einer der unter B1) genannten Verbindung enthalten.

7. Perlglanzdispersion nach einem oder mehreren der Ansprüche 1 bis 3, dadurch gekennzeichnet, daß sie frei von mehrwertigen C₂-C₆-Alkoholen sind und mehr als 10 bis 30 Gew.-% einer der unter B1) genannten Verbindungen enthalten.

8. Perlglanzdispersion nach einem oder mehreren der Ansprüche 1 bis 7, dadurch gekennzeichnet, daß sie Konservierungsmittel und/oder Puffersubstanzen enthält.

9. Perlglanzdispersion nach einem oder mehreren der Ansprüche 1 bis 5, dadurch gekennzeichnet, daß sie bis zu 10 Gew.-% an mehrwertigen C₂- bis C₈-Alkoholen enthält.

10. Perlglanzdispersion nach Anspruch 9, dadurch gekennzeichnet, daß mehrwertige C₂- bis C₆-Alkohole verwendet werden.

11. Perlglanzdispersion nach Anspruch 9 oder 10, dadurch gekennzeichnet, daß als mehrwertige Alkohole Ethylenglykol, 1,2-Propylenglykol, 1,3-Propylenglykol, Glycerin, Diethylenglykol, Triethylenglykol, Erythrit, Arabit, Adonit, Xylit, Sorbit, Mannit, Dulcit oder Mischungen daraus verwendet werden.

12. Tensidformulierung, enthaltend 1 bis 10 Gew.-% einer Perlglanzdispersion gemäß einem oder mehrerer der Ansprüche 1 bis 11.

13. Flüssige Feinwaschmittel, Universalwaschmittel, Handgeschirrspülmittel, Klarspüler, flüssige Reinigungs- und Desinfektionsmittel, flüssige Seifen, Haarshampoos, Haarspülungen, Haarfärbemittel, Haarwellmittel, Schaumbäder, Gesichtsreinigungsmittel, Duschbadzubereitungen und 2 in 1-Formulierungen, dadurch gekennzeichnet, daß sie eine Perlglanzdispersion gemäß einem oder mehrerer der Ansprüche 1 bis 11 enthalten.
